Europäisches Patentamt

European Patent Office

Office européen des brevets

(11) Publication number: **0 300 682**
**A1**

## EUROPEAN PATENT APPLICATION

(21) Application number: 88306427.1

(22) Date of filing: **13.07.88**

(51) Int. Cl.⁴: **G01N 33/58 , C12Q 1/68 , G01N 33/74 , G01N 33/53**

(30) Priority: **13.07.87 US 72463**

(43) Date of publication of application:
**25.01.89 Bulletin 89/04**

(84) Designated Contracting States:
**BE DE FR GB IT NL SE**

(71) Applicant: **THE UNIVERSITY OF TENNESSEE RESEARCH CORPORATION**
**Suite 415, Communications Building**
**Knoxville, TN 37966 - 0344(US)**

(72) Inventor: **Huang, Leaf**
**352, Dominion Circle**
**Knoxville Tennessee, 37922(US)**

(74) Representative: **Davies, Jonathan Mark et al**
**Reddie & Grose 16 Theobalds Road**
**London WC1X 8PL(GB)**

(54) A homogeneous, lipsome-based signal amplification method for assays involving enzymes.

(57) The present invention is directed to a procedure for enhancing the enzyme-dependent readout of diagnostic assays using specially prepared liposomes.

Microgenic's CEDIA assay for digoxin was employed as an illustrative example of the present invention. The magnitude of signal was only 25 mA/min/ng digoxin/ml for the unamplified assay, while the signal magnitude for the amplified (i.e., liposome-enhanced) assay was 1,000 mA/min/ng digoxin/mL, i.e., a 40-fold amplification.

Stable liposomes were prepared with unsaturated PE stabilized with 5 mole percent of ganglioside GM₁. Glucose-6-phosphate dehydrogenase (G6PHD) was entrapped in these unilamellar·liposomes in high concentrations. Addition of beta-galactosidase (B-gal) caused rapid (3-5 min.) lysis of liposomes revealing the latent G6PDH activity, due to the enzymatic degalactosylation of MG₁.

This simple, rapid and homogenous signal amplification procedure will be useful in many enzyme dependent assays, such as ELISA, CEDIA, Gene-probe assays and Immunoliposome assays.

FIG. I

# A HOMOGENEOUS, LIPOSOME-BASED SIGNAL AMPLIFICATION METHOD FOR ASSAYS INVOLVING ENZYMES

## BACKGROUND OF THE INVENTION

Many phospholipids can be dispersed in aqueous media to yield closed bilayer vesicles (liposomes) which can be unilamellar or multilamellar structures. However, non-bilayer structures such as micelle or hexagonal ($H_{II}$) phases can exist in many phospholipid and glycolipid systems.

It has been established that the equilibrium phase of unsaturated phosphatidyl ethanolamine (PE) at physiological temperature and pH is the hexagonal phase ($H_{II}$) (Cullis, et al., Biochem. Biophys. Acta, 559, 399-420 (1979)).

It has further been established that PE can be stabilized in bilayer form by using various ampipathic stabilizer molecules such as fatty acids (Connor et al., Cancer Res., 46, 3431-3443 (1986)); cholesteryl hemisuccinate (Ellens et al., Biochemistry, 25, 5500-5506 (1984)); haptenated lipids (Ho et al., J. Immunol., 134, 4035-4040 (1985)); glycophorin (Ho et al., J. Immunol., 134, 4035-4040 (1985); Taraschi et al., Biochemistry, 21, 5756-5764 (1982); Hu et al., Biochem. Biophys. Res. Commun., 141, 973-978 (1986)); gangliosides (Tsao et al., Biochemistry, 24, 1092-1098 (1985)); and acylated antibody (Ho et al., Biochemistry, 25, 5500-5506 (1986)). Antibody stabilized PE liposomes, named target-sensitive immunoliposomes (Ho et al., Biochemistry, 25, 5500-5506 (1986); Ho et al., Biochem. Biophys. Res. Commun., 138, 931-937 (1986)), have been shown to release their liposomal contents upon binding to a specific target.

Liposome lysis is independent of complement or any other lytic agents. It has been used to construct a homogeneous assay for the Herpes Simplex virus (Ho et al., Biochem. Biophys. Res. Commun., 138, 931-937 (1986)).

Due to the fact that a large quantity of enzyme molecules can be entrapped in each liposome vesicle, a liposome-based assay is generally more sensitive than the corresponding enzyme immunoassay or radioimmunoassy (D. Monroe, American Clinical Products Review. December issue pp. 34-41 (1986)). However, some analytes are present in such low concentrations that even the liposome-based assays of current design are inadequate for detection.

The present invention is directed to a novel use of stabilized $H_{II}$ phase forming liposomes for signal enhancement in homogeneous assays involving enzymes. This invention is applicable to many assays requiring sensitivity enhancement.

## SUMMARY OF THE INVENTION

It has been shown that the enzymatic degradation of a stabilizer compound present in stabilized $H_{II}$ phase lipid containing liposomes, leads to the rapid destabilization (or lysis) of the stabilized liposomes.

For example, removal by papain of the Fab domain of the antibody on a target-sensitive immunoliposome causes destabilization of the otherwise stable liposome (Ho et al., Biochemistry, 25, 5500-5506 (1986)). The bilayers of DOPE-glycophorin liposomes are also destabilized when the stabilizer glycophorin on the liposome was proteolytically cleaved by trypsin (Hu et al., Biochem. Biophys. Res. Commun., 141, 973-978 (1986)). Thus, an enzymatic cleavage of the hydrophilic portion of the stabilizer caused the destabilization of the otherwise stable liposome.

Relying upon these principles, the present inventor designed a liposome mediated signal enhancement procedure which takes advantage of the fact that an $H_{II}$ phase forming lipid (e.g., phosphatidyl ethanolamine or PE) can be stabilized with one or more stabilizer compounds or molecules as described supra (e.g., with ganglioside $GM_1$).

The present invention requires that the stabilizer compound be a substrate for an enzyme employed in any of the enzymatic assays currently available, (e.g., $GM_1$ is a substrate for the beta-galactosidase enzyme or B-gal).

In addition, the present invention requires that the product of the enzymatic reaction between the stabilizer compound and the assay enzyme no longer acts as a stabilizer for the liposome composition, i.e., for the present invention to operate, the liposomes must lyse.

The present invention also requires that the liposomes be prepared with an entrapped reporter enzyme or other easily detected reporter species (e.g., one useful reporter enzyme is glucose-6-phosphate dehydrogenase or G6PDH).

Thus, in an illustrative embodiment, free (active) B-gal which may be released from either a liposome, due to analyte dependent immune lysis (Ho et al., Liposome as drug carriers; Trends and Progress, G. Gregoriadis (in press) (1987)), or is reconstituted from inactive components such as those used in the CEDIA assay (Henderson et al., Clin. Chem., 32, 1637-1641 (1986)), or is part of enzyme - antibody or enzyme - avidin conjugates, such as in ELISA or gene-probe assays, attacks the $GM_1$ on the liposomes of the present invention, which are added to the assay system. The thus destabilized liposomes release the entrapped reporter enzyme, providing an enhanced readout signal.


## BRIEF DESCRIPTION OF THE DRAWINGS


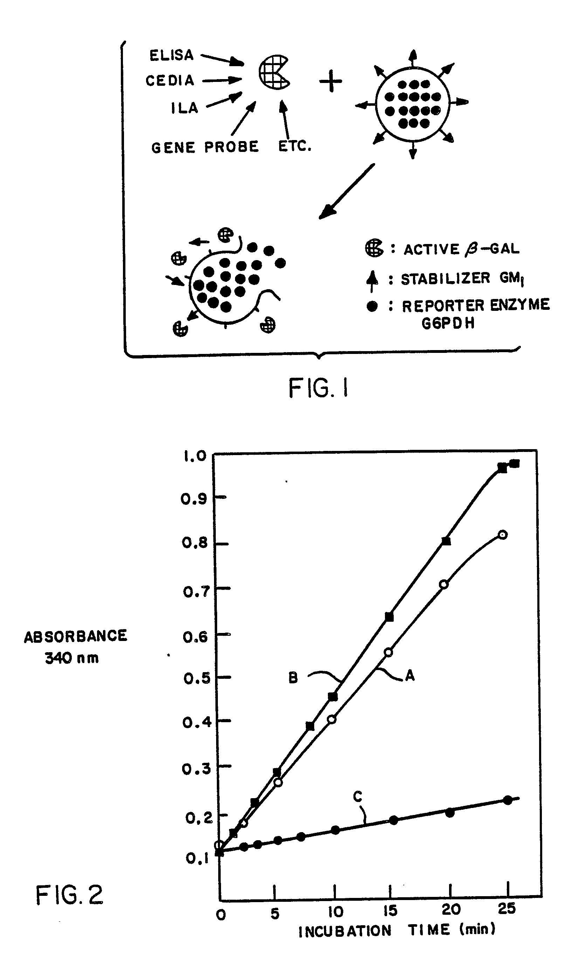

Fig. 1 illustrates, by means of a preferred embodiment, the principles of the liposome-mediated signal enhancement procedure of the present invention. An active enzyme (e.g., B-gal) which was either reconstituted or released in an analyte-dependent manner, degrades a liposome-stabilizer (e.g., $GM_1$), causing the lysis of the stabilized liposomes and the release of an entrapped reporter enzyme (e.g., G6PDH); thus amplifying the analyte-induced signal.

Fig. 2 is a plot showing the release of G6PDH enzyme from liposomes. The liposomes (100 micro-M were preincubated with B-gal (2 mg/ml) (A) or 0.15% Triton X-100 (B) or buffer (C) for 10 minutes at 37°. The resulting reaction mixture was diluted to a final liposome concentration of 20 micro-M, substrates for G6PDH was added and the enzyme activity was measured over a 30 minute time period. The measurements were done in triplicate and standard deviation was ± 6%.

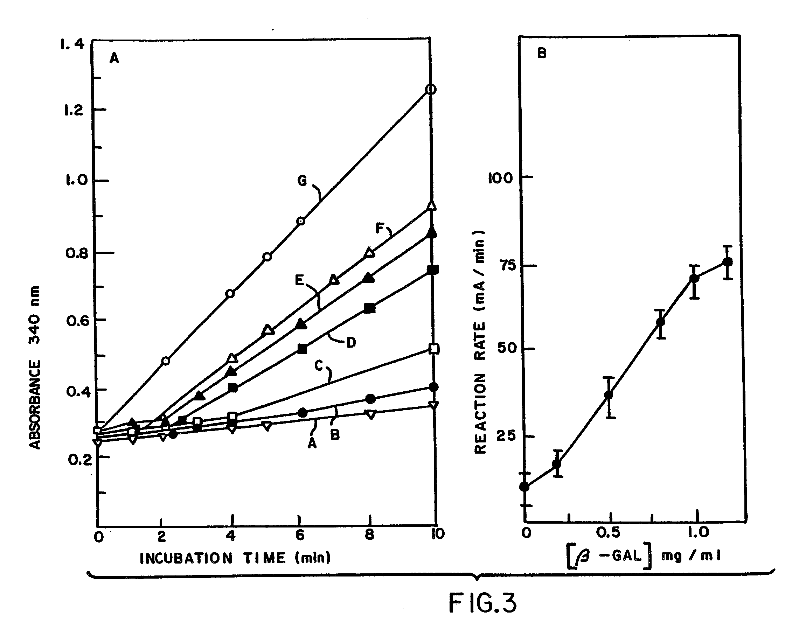

Fig. 3(A) is a plot showing the B-gal induced release of G6PDH from $GM_1$-PE liposomes. Liposomes (50 micro-M), G6P (7 mM) and $NAD^+$ (2 mM) were (0 - 1.2 mg/ml) at 37° and the reaction was followed by monitoring the absorbance at 340 nm over a 10 minute time period. Liposomes in the presence of 0 mg/ml (A), 0.2 mg/ml (B), 0.5 mg/ml (C), 0.8 mg/ml (D), 1.0 mg/ml (E) or 1.2 mg/ml (F) of beta-galactosidase, or in the presence of 0.15% Triton X-100 (G).

Fig. 3(B) is a plot showing the rate of G6PDH catalyzed reaction as a function of the B-gal enzyme concentration.

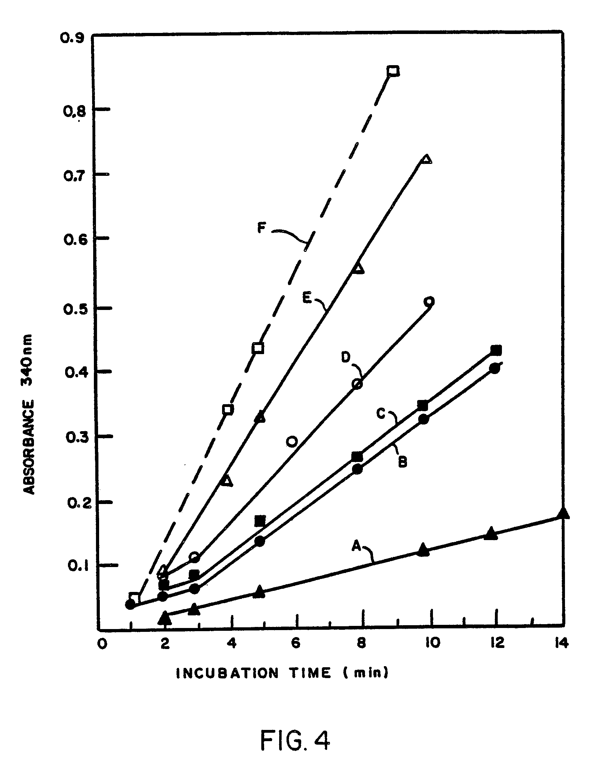

Fig. 4 is a plot illustrating the liposome enhanced CEDIA assay at different digoxin concentrations. The activity of G6PD was monitored by following the absorbance at 340 nm. The plots illustrated are: (A), liposomes in PBS; (B), liposomes in the presence of digoxin at 0 ng/ml; (C), digoxin at 0.01 ng/ml; (D), digoxin at 0.02 ng/ml; (E), digoxin at 0.04 ng/ml; and (F), digoxin at 0.06 ng/ml. The measurements were done in quadruplicates.

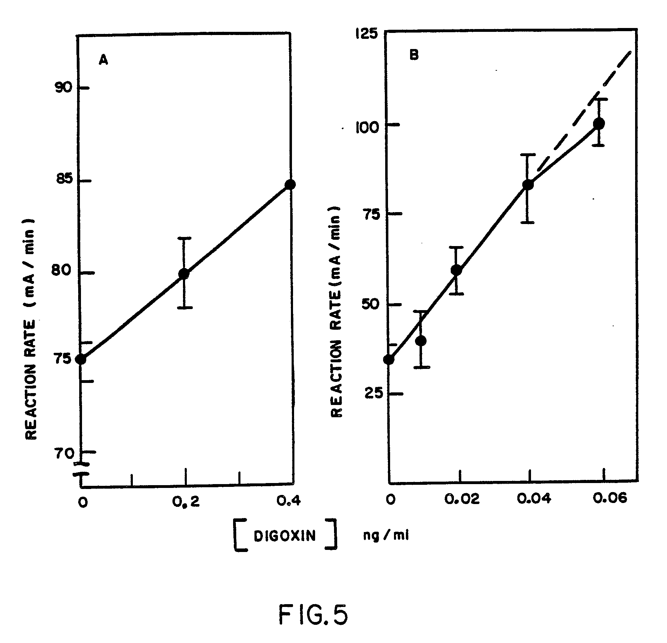

Fig. 5(A) is a plot of a standard CEDIA Digoxin assay using o-nitrophenyl-D-galactopyranoside ONPG as the substrate. The assay was performed according to manufacturer's recommendations. The measurements were done in quadruplicate.

Fig. 5(B) illustrates the increased rate of G6PDH catalysed reaction in the liposome-enhanced CEDIA assay as a function of digoxin concentration in the serum. Measurements were done in quadruplicate. These data were derived from Figure 4.


## DETAILED DESCRIPTION OF THE PREFERRED EMBODIMENTS


Taking advantage of the fact that liposomes formed from $H_{II}$-phase forming lipids, such as phosphatidyl -ethanolamine can be stabilized by a membrane lipid or protein, and that the destruction of the stabilizer leads to a rapid lysis of the liposomes, the present inventor has designed a liposome-based signal enhancement procedure for assays which involve an enzyme in the final read-out step.

In an illustrative embodiment, stable liposomes were prepared with unsaturated phosphatidylethanolamine (PE) stabilized with 5 mole percent of ganglioside $GM_1$. Glucose-6-phosphate dehydrogenase (G6PDH) was entrapped in these unilamellar liposomes in high concentrations. The addition of beta-galactosidase (B-gal) caused rapid (3-5 min.) lysis of these liposomes revealing the latent G6PDH activity, due to the enzymatic degalactosylation of $GM_1$.

Since each molecule of B-gal can destroy many liposomes and since a large quantity of the reporter enzyme, G6PDH, can be released from each liposome, the magnitude of signal amplification is large.

The present inventor employed Microgenic's CEDIA assay for digoxin as an illustrative example for the procedure of the present invention. By way of comparison, the magnitude of the assay signal was 25 mA/min/ng digoxin/ml in a standard CEDIA assay, while the signal magnitude for the amplified (i.e., liposome-enhanced) assay was 1,000 mA/min/ng digoxin/ml, i.e., a 40-fold amplification.

The liposomes used for signal enhancement were stable in the presencce of 10% human serum throughout the assay period. This simple, rapid and homogeneous signal amplification method will be useful in many enzyme dependent assays, such as ELISA, CEDIA, Gene-probe assays and Immunoliposome assays, and the like.

In the CEDIA assay, the enzyme B-galactosidase is divided into two, enzymatically inactive components $E_d$ and $E_a$; which spontaneously reconstitute to give the active enzyme. One of the components, E is covalently linked with an analyte such that the antibody bound $E_d$ can no longer participate in the enzyme reconstitution. In the presence of free analyte in a test specimen, $E_d$ is not bound by antibody and active enzyme molecules are reconstituted.

In the illustrative embodiment of the present invention, the analyte-antibody reaction controls the formation of the active enzyme which in turn determines the fate of the stabilizer $GM_1$ on the PE-liposomes. Degalactosylation of the $GM_1$ on the surface bilayer of the PE liposome causes destabilization of the liposome releasing the reporter enzyme molecule, resulting in an amplified signal (see, Fig. 1).

The liposomes of the present invention were prepared by the fast extrusion method of R.J. Ho, Ph.D. Dissertation, (1987 Univ. of Tenn., Knoxville. A liposome composition of dioleylphosphatidylethanolamine : transphosphorylated phosphatidylethanolamine (DOPE: TPE) (2:1 M/M) with 5 mole % $GM_1$ was preferred.

After preparing the liposomes, nonentrapped G6PDH was removed by Sepharose 4B column chromatography. Lipsomes labeled with a trace amount of tritiated hexadecyl cholestanylether ($[^3H]$-CE) were eluted in the void volume. Even after this purification step, some free (non-latent) enzyme activity was usually detected (8-12% of the total activity), which may be due to a weak association of the enzyme with the outer surface of the bilayer membrane.

G6PDH enzyme activity was measured by continuous monitoring of absorbance of 340 nm. As shown in Fig. 2, there was about 10% of the total enzyme activity in the intact liposome preparation. However, the majority of G6PDH was latent and could be released by disrupting the membrane with 0.15% Triton X-100.

B-Galactosidase (B-gal) pretreated liposomes released about 80% of the encapsulated G6PDH compared to that of Triton-pretreated liposomes. These data show that the enzymatic action of B-galactosidase on $GM_1$ of the liposomes causes the otherwise stable liposomes to release their contents.

However, the B-gal treatment of the $GM_1$-PE liposomes maximally released about 85%, never 100% of the encapsulated enzyme. It is possible that the degalactosylated $GM_1$ ($GM_2$) was still able to partially stabilize the bilayer phase of the PE.

This result agrees with an earlier observation that trypsin treatment of the glycophorin-PE liposomes released only about 70% of the encapsulated marker (Hu et al., Biochem, Biophys. Res. Commun., 141, 973-978 (1986)).

Control liposomes composed of 5 mole % $GM_1$ in egg phosphatidylcholine (PC) were stable after treatment with B-gal. This is because unlike PE, the PC bilayer is stable even after the enzymatic degradation of $GM_1$.

Referring to Figures 3(A) and 3(B), the B-gal concentration dependence on the lysis of $GM_1$-PE liposomes was measured at 37°C for up to 10 minutes. These experiments were done with liposomes, G6PDH substrates, and B-gal added at the same time, i.e., with no preincubation of the liposomes with B-gal as was done in the experiments shown in Fig. 2.

As illustrated in Fig. 3(A), at low enzyme concentrations, there was an initial lag period followed by a rapid increase of absorbance at 340 nm. This lag period could be shortened by increasing the concentration of the B-gal present in solution.

Cleavage of substantial fraction of $GM_1$ may be necessary to cause the destabilization of the liposome. Since the enzyme concentration is limiting, it may require some time to cleave sufficient numbers of GM molecules of the liposome to make is unstable, hence resulting in a lag period. On the other hand, the stable enzymatic reaction rate was enhanced with higher B-gal concentrations.

Fig. 3(B) shows that the rate of release of G6PDH from the liposomes increases linearly with increasing B-gal concentration. The slope of the linear portion of the curve is 84 mA/min/mg B-gal/ml.

The stability of the $GM_1$-PE liposomes was determined in the presence of different concentrations of human serum (see Table 1).

## Table 1.

### Serum stability of $GM_1$-PE liposomes[a]

| % serum in PBS (pH 7.2) | EGTA Conc. mM | G6PDH activity (% latent activity)[b] |
|---|---|---|
| 100 | 0 | 0 |
| | 2 | 13 |
| | 5 | 18 |
| 50 | 0 | 80 |
| | 3 | 99 |
| 20 | 0 | 97 |
| | 2 | 100 |
| 10 | 0 | 99 |
| | 2 | 100 |
| 0 | 0 | 100 |
| | 2 | 100 |

---

(a) Liposomes (50 micro-M) were incubated with different concentrations of serum in the presence or absence of EGTA for 5 minutes in the cuvette. The % latent G6PDH activity in the resulting mixture was taken as an indicator of the stability of liposomes.

(b) % latent activity =

$$[1 - (\frac{\text{activity in serum} - \text{activity in PBS}}{\text{activity in Triton} - \text{activity in PBS}})] \times 100$$

Although the liposomes were not stable in neat serum, they were fairly stable in up to 50% serum solution. It should be noted that the presence of 2 mM or more EGTA greatly enhanced the stability of these liposomes in serum, indicating that removal of divalent cations such as $Ca^{++}$ enhanced liposome stability.

It has been noted previously that liposomes composed of PE are more stable in serum compared to those composed of PC. Liposome stability in serum can be significantly improved by addition of cholesterol (Kirby et al., Biochem. J., 186, 591-598 (1980) and Damen et al., Biochim. Biophys. Acta., 665, 538-545 (1981)) and/or gangliosides (Allen et al., Biochim. Biophys. Acta., 818, 205-210 (1985)). Apparently, it is the presence of 5 mole % $GM_1$ in PE makes these liposomes relatively stable in serum.

The use of the $GM_1$ stabilized PE liposomes containing G6PDH to enhance the sensitivity of a homogeneous enzyme reconstitution immunoassay is described below.

The CEDIA digoxin assay was selected as a model assay since this assay uses the reconstitution of B-galactosidase enzyme (B-gal) in an analyte-dependent manner as a read-out. The amount of active B-gal formed is thus proportional to the amount of digoxin present in a patient's serum.

Initially, different concentrations of liposomes were studied to determine the saturation concentration of liposomes to be used in subsequent experiments. Increasing liposome concentration resulted in an increase of the G6PDH activity up to 40 micro-M of lipid. Thereafter, 50 micro-M of liposomes were used for subsequent assays.

As can be seen in Figure 4, there was a digoxin concentration-dependent increase of liposome lysis as evidenced by the enhanced rate of absorbance at 340 nm.

As described above, there was a lag period (2 - 3 min.) prior to the maximal enzymatic rate being established. The enzymatic rate was stable for at least 8 to 10 minutes, generating an absorbance (340 nm) reading of 0.35 to 0.90, carried out using different batches of liposome preparations. Intra- and interassay precision were within 8%.

The results of a liposome-enhanced CEDIA assay are compared with those of the original unenhanced assay (see, Figs. 5(A) and (B)). The original CEDIA assay was carried out according to manufacturer's instructions. As can be seen in these Figures, the original CEDIA assay for digoxin gave rise to a linear response to digoxin concentration in the range 0-0.4 ng/ml in agreement with manufacturer's specifications.

The sensitivity of the assay (the slope of the standard curve) was 25 mA/min/ng digoxin/ml (see, Fig. 5-(A)). Similarly, the liposome-enhanced assay also gave rise to a linear response up to 0.04 ng/ml with saturation occurring at 0.06 ng/ml. The sensitivity of the enhanced assay (the slope of the curve) was 1,000 mA/min/ng digoxin/ml (see, Fig. 5(B)). There was thus a 40-fold enhancement of the magnitude of the signal. The lowest concentration of digoxin which still gave a statistically significant different ($P < 0.05$, student t-test) from that of zero digoxin concentration was 0.4 ng/ml (0.52 pmoles/ml) and 0.02 ng/ml (0.026 pmoles/ml) for the original and liposome-enhanced assays, respectively.

Digoxin concentrations of 0.09 to 2.0 ng/ml in serum are normally considered to be therapeutic (Beller et al., New Engl. J. Med., 284, 929-997 (1971) and Lindenbaum et al., Lancet, 1, 1215-1217 (1973)). Generally, the symptoms of toxicity only appear at concentrations above 2 ng/ml (Beller et al., New Engl. J. Med., 284 929-997 (1971) and Butler et al., Am. J.

Although it is not necessary to increase the sensitivity of the CEDIA assay for digoxin, the present liposome-based enhancement will certainly be useful in increasing the sensitivity of many immunochemical assays based on the same principle of design described herein. See for example, Amshey, "Enzyme Immunoassay," Ann. Rpts. Med. Chem., 18, 285-292 (1983), which is hereby incorporated herein by reference.

For example, the assay for thyroid stimulating hormone (TSH) requires a sensitivity as low as 0.02 pmoles/ml (R. J. Kitsky, Handbook of Vitamins, Minerals and Hormones, 2nd Edition, VNR Company, NY (1981) and Rosenfield et al., Clin. Chem., 32, 232-233, (1986)). The liposome-based signal amplification procedure of the present invention would be uniquely suitable to achieve the necessary degree of high sensitivity required for this assay. Furthermore, the enhancement step is homogeneous, requiring no washing or separation steps. Substrates and liposomes could be added together and the total incubation time only increases nominally.

Finally, this amplification procedure will be useful in enhancing the sensitivities of many other non-isotopic assays which involve enzyme as the final readout step, such as ELISA, immunoliposome assays and gene-probe assays, and the like.

## EXAMPLES

The present invention will be further illustrated with reference to the following examples which aid in the understanding of the present invention, but which are not to be construed as limitations thereof. All percentages reported herein, unless otherwise specified, are percent by weight. All temperatures are expressed in degrees Celsius.

## MATERIALS

TPE [phosphatidylethanolamine (PE) which is transphosphatidylated from egg phosphatidylcholine (PC)-], dioleoyl PE (DOPE), and egg PE were purchased from Avanti Polar Lipids (Birmingham, AL). Ganglioside $GM_1$ was obtained from Supelco (Houston, TX) or Calbiochem (San Diego, CA). The purity of $GM_1$ was confirmed with TLC using chloroform / metanol / 2.5N ammonium hydroxide (60:40:9) as a developing system (Skipski, Methods in Enzymology, 35, 396-425 (1975) and Kundu, Methods in Enzymology, 72, 185-204 (1981)). Tritiated [$^3$H] hexadecyl cholestanyl ether was synthesized and purified as described by Pool et al., Lipids, 17, 448-452 (1982).

Glucose - 6 - phosphate dehydrogenase (G6PDH), glucose-6-phosphate (G6P) and nicotinamide dinucleotide ($NAD^+$) were purchased from Boehringer Mannheim (Indianapolis, IN). B-galactosidase and its substrate, o-nitrophenyl- D-galactopyranoside (ONPG) were obtained from Sigma Chemical Co. (St. Louis, MO). The CEDIA Digoxin assay kit was obtained from Microgenics Corp. (Concord, CA). All other compounds were of analytical grade.

## EXAMPLE 1

## LIPOSOME PREPARATION

Liposomes were prepared by the fast extrusion method of R.J. Ho, Ph.D. Dissertation, (1987) Univ. of Tenn., Knoxville. A liposome composition of DOPE:TPE (2:1 M/M) with 5 mole % $GM_1$ was used. Routinely, 3 mole DOPE and 1.5 mole TPE and 0.23 mole $GM_1$ mixed with a trace amount of [$^3$H]-CE (1 X 10$^9$ cpm/mole lipid) were dried under a stream of $N_2$ and vacuum desiccated for no less than one hour.

The lipid mixture was next hydrated with the enzyme G6PDH (2000 units) in PBS (total volume of 500 micro-1, pH 7.6) containing 10 mM G6P (15) (Canova-Davis, et al., Clin. Chem., 32, 1687-1691 (1986)), 1 mM EGTA and 0.02% $NaN_3$. The mixture was allowed to stand at room temperature for 2-3 hours with occasional mixing. The lipid mixture was then vortexed and allowed to stand at room temperature for an additional hour.

Next, the suspension was extruded quickly and forcefully through a 28-gauge needle using a 0.5 ml B-D insulin syringe (20 times). Two additional cycles of extrusion were performed with a 4-12 hour resting interval.

The negative stained electron micrographs showed that the resulting liposomes were mainly unilameller with an average diameter of 1500 Angstroms.

Non-entrapped enzyme was removed by chromatography on a Sepharose 4B column with a flow rate of 0.4 ml/min. The fractions were measured for $^3$H-cpm and G6PDH activity in the presence or absence of 0.15% Triton X-100. The peak samples were pooled and stored at 4° C at a lipid concentration of 500 micro-M. Such liposomes were stable in physiological saline at 4° C for at least 3 weeks. Approximately 5% of G6PDH was trapped in liposomes.

The stock liposomes (hereinafter called $GM_1$-PE liposomes) were diluted to an appropriate lipid concentration immediately before each assay.

## EXAMPLE 2

## ASSAY FOR G6PDH

Enzyme activity was measured by incubating the buffer (3 mM $Mg^{++}$), pH 7.6 or PBS, pH 7.6 containing 7 mM G6P and 2 mM $NAD^+$; at room temperature or 37° C (Canova-Davis et al., Clin. Chem., 32, 1687-1691 (1986)).

Total enzyme activity in the liposome preparation was assayed after disrupting the liposomes with 0.15% Triton X-100. The reaction was followed by monitoring the absorbance at 340 nm using a Perkin Elmer Model 3A spectrophotometer equipped with a water-jacketed cell holder.

EXAMPLE 3

B-GAL INDUCED RELEASE OF G6PDH FROM LIPOSOMES

Liposomes (80-100 micro-M lipid) containing entrapped G6PDH were preincubated for 10 minutes at 37°C with B-gal (2 mg/ml) or 0.15% Triton before the addition of substrates, G6P and NAD$^+$. The temperature of the cell holder was controlled by a circulating water bath with a variation of ± 0.3°C. The release of G6PDH was measured by monitoring the absorbance change at 340 nm.

EXAMPLE 4

DIGOXIN ASSAY USING B-GAL AS FINAL READOUT STEP

The CEDIA digoxin assay kit was used following the manufacturer's instructions, except that the reaction at 37°C was continuously monitored for absorbance at 420 nm.

EXAMPLE 5

LIPOSOME-COUPLED ENZYME RECONSTITUTION ASSAY

The CEDIA Digoxin assay reagents were reconstituted and used according to manufacturer's recommendations, except that the $E_d$ reagent was dialyzed overnight at 4°C, against 0.1 M sodium phosphate buffer containing 1 mM MgCl$_2$, pH 7.2 to remove the endogenous substrate which interfered with the subsequent assay of G6PDH.

The liposome-enhanced enzyme assay was performed as follows. The $E_a$ (60 micro-1) and the sample (25 micro-1 in human serum) were incubated at 37°C for 4 minutes with occasional mixing. The dialyzed $E_d$ reagent (37.5 micro-1) was then added and incubation continued for 4 more minutes. Finally, the liposomes were added together with the substrates G6P (7 mM) and NAD$^+$ (2 mM) and the absorbance at 340 nm was monitored for 10-15 minutes. The total reaction volume was 250 micro-1.

The present invention has been described in detail, including the preferred embodiments thereof. However, it will be appreciated that those skilled in the art, upon consideration of the present disclosure, may make modifications and/or improvements on this invention and still be within the scope and spirit of this invention as set forth in the following claims.

## Claims

1. A method for the liposome-mediated signal enhancement of diagnostic assays systems involving an enzyme in the readout step comprising:

(a) selecting an enzyme-based diagnostic assay system, utilizing an enzyme which is retained, released, reconstituted, or activated in an analyte-dependent manner in a test fluid;

(b) providing a liposome prepared from an otherwise unstable $H_{II}$ phase forming molecule, together with an effective amount of an ampipathic liposome stabilizer compound, said liposomes having trapped therein one or more reporter enzymes and wherein said stabilizer compound is a substrate for the enzyme in (a);

(c) combining the enzyme in (a) with the liposomes of (b), such that reaction between the enzyme of (a) and the stabilizer compound for the liposomes of (b) causes lysis of the liposomes and release of the entrapped reporter enzyme; and

(d) adding an appropriate amount of a substrate for the released reporter enzyme; which substrate does not permeate the intact liposome membrane; such that the combination of released reporter enzyme and substrate therefor acts to enhance the readout signal of the assay system.

2. The method of claim 1, wherein the steps (c) and (d) are performed substantially simultaneously.

3. The method of claim 1, wherein the enzyme based diagnostic assay system is selected from the group consisting of EIA, ELISA, EMIT, SLFIA, CELIA, CEDIA, ELA, TELISA, ELIA, IEIA, immunochemical assays, immunoliposome assays, and gene-probe assays.

4. The method of claim 1, wherein the stabilizer compound is selected from the group consisting of fatty acids, cholesteryl hemisuccinate, haptenated lipids, gangliosides, glycoproteins, proteins, acylated antibodies and nucleic acids,.

5. The method of claim 4, wherein the ganglioside stabilizer compound is $GM_1$.

6. The method of claim 1, wherein the $H_{II}$ phase forming molecule is a lipid.

7. The method of claim 6, wherein the $H_{II}$ phase forming lipid is a monoglycosyl diglyceride.

8. The method of claim 6, wherein the $H_{II}$ phase forming lipid is one or more phosphatidylethanolamines.

9. The method of claim 8, wherein the phosphatidylethanolamine comprises a mixture of DOPE and TPE in a mole ratio of 2:1.

10. The method of claim 8, wherein the $H_{II}$ phase forming lipid is unsaturated.

11. The method of claim 1, wherein the effective amount of ampipathic stabilizer compound is up to about 5 mole percent.

12. The method of claim 1, wherein the enzyme useful in a diagnostic enzyme assay is selected from the group consisting of beta-galactosidase, horse radish peroxidase, alkali phosphatase, glucose-6-phosphate dehydrogenase and glucose oxidase.

13. The method of claim 1, wherein the enzyme based diagnostic assay is a thyroid stimulating hormone assay.

14. The method of claim 1, wherein the enzyme based diagnostic assay is the CEDIA assay for digoxin.

15. The method of claim 14, wherein the liposomes comprise DOPE:TPE.

16. The method of claim 14, wherein the stabilizer compound is ganglioside $GM_1$.

17. The method of claim 14, wherein the reporter enzyme is glucose-6-phosphate dehydrogenase.

18. Liposomes comprising one or more unstable $H_{II}$ phase forming lipids which will not form liposomes per se and a sufficient quantity of an ampipathic stabilizer compound, said liposomes having entrapped therein, one or more reporter enzymes, and wherein said stabilizer compound is a substrate for an enzyme useful in diagnostic enzyme assay systems.

19. The liposomes of claim 18, wherein the stabilizer compound is selected from the group consisting of fatty acids, cholesteryl hemisuccinate, haptenated lipids, gangliosides, glycoproteins, proteins, acylated antibodies, and nucleic acids.

20. The liposomes of claim 19, wherein the liposome stabilizer compound is ganglioside $GM_1$.

21. The liposomes of claim 18, wherein the $H_{II}$ phase forming lipid is a monoglycosyl diglyceride.

22. The liposomes of claim 18, wherein the $H_{II}$ phase forming lipid is a phosphatidylethanolamine.

23. The liposomes of claim 22, wherein the phosphatidylethanolamine comprise a mixture of DOPE and TPE in a mole ratio of 2:1.

24. The liposomes of claim 18, wherein the $H_{II}$ phase forming lipid is unsaturated.

25. The liposomes of claim 18, wherein the sufficient quantity of ampipathic stabilizer compound is up to about 5 mole percent.

26. The liposomes of claim 18, wherein the enzyme based diagnostic assay is a thyroid stimulating hormone assay.

27. The liposomes of claim 18, wherein the enzyme based diagnostic assay is the CEDIA assay for digoxin.

28. The liposomes of claim 27, wherein the liposomes comprise DOPE:TPE.

29. The liposomes of claim 27, wherein the stabilizer compound is ganglioside $GM_1$.

30. The liposomes of claim 27, wherein the reporter enzyme is glucose-6-phosphate dehydrogenase.

31. The liposomes of claim 18, wherein the enzyme useful in a diagnostic enzyme assay is selected from the group consisting of beta-galactosidase, horse radish peroxidase, alkali phosphatase, glucose-6-phosphate dehydrogenase and glucose oxidase.

FIG. 1

FIG. 2

FIG.3

EP 0 300 682 A1

FIG. 4

FIG.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| P,A | WO-A-8 704 795 (UNIVERSITY OF TENNESSEE RESEARCH CORP.) --- | | G 01 N 33/58<br>C 12 Q 1/68<br>G 01 N 33/74<br>G 01 N 33/53 |
| A | EP-A-0 138 685 (THE LIPOSOME CO., INC.) --- | | |
| A | WO-A-8 604 682 (COOPER-LIPOTECH, INC.) --- | | |
| A | EP-A-0 174 753 (BECTON, DICKINSON AND CO.) --- | | |
| A | GB-A-1 575 344 (IMPERIAL CHEMICAL INDUSTRIES LTD) --- | | |
| D,A | BIOCHEMISTRY, vol. 24, no. 5, 1985, pages 1092-1098, American Chemical Society; Y.-S. TSAO et al.: "Sendai virus induced leakage of liposomes containing gangliosides" --- | | |
| A | BIOCHEMISTRY, vol. 24, no. 7, 1985, pages 1654-1661, American Chemical Society; M.-Z. LAI et al.: "Acid- and calcium-induced structural changes in phosphatidylethanolamine membranes stabilized by cholestryl hemisuccinate" ----- | | TECHNICAL FIELDS SEARCHED (Int. Cl.4)<br><br>G 01 N<br>A 61 K |

The present search report has been drawn up for all claims

| Place of search | Date of completion of the search | Examiner |
|---|---|---|
| THE HAGUE | 18-10-1988 | CARTAGENA Y ABELLA P. |